(19) **Europäisches Patentamt / European Patent Office / Office européen des brevets**

(11) **EP 1 755 552 B1**

(12) **EUROPEAN PATENT SPECIFICATION**

(45) Date of publication and mention
of the grant of the patent:
**24.10.2012 Bulletin 2012/43**

(51) Int Cl.:
*A61Q 11/00* (2006.01)    *A61K 8/81* (2006.01)
*A61K 8/02* (2006.01)    *A61K 9/00* (2006.01)

(21) Application number: **05760577.6**

(22) Date of filing: **16.06.2005**

(86) International application number:
**PCT/US2005/021129**

(87) International publication number:
**WO 2006/009737 (26.01.2006 Gazette 2006/04)**

(54) **ORAL CARE FILM**

MUNDPFLEGEFILM

FILM D'HYGIENE BUCCALE

(84) Designated Contracting States:
**AT BE BG CH CY CZ DE DK EE ES FI FR GB GR HU IE IS IT LI LT LU MC NL PL PT RO SE SI SK TR**

(30) Priority: **17.06.2004 US 870848**

(43) Date of publication of application:
**28.02.2007 Bulletin 2007/09**

(73) Proprietor: **Colgate-Palmolive Company New York NY 10022-7499 (US)**

(72) Inventors:
• **IBRAHIM, Sayed**
  **Somerset, New Jersey 08873 (US)**

• **CHOPRA, Suman, K.,**
  **Dayton, New Jersey 08810 (US)**
• **PRENCIPE, Michael**
  **West Windsor, New Jersey 08550 (US)**

(74) Representative: **Jenkins, Peter David et al Page White & Farrer Bedford House John Street London WC1N 2BF (GB)**

(56) References cited:
WO-A-01/34121        WO-A-03/000216
DE-A1- 19 652 268    DE-A1- 19 745 208
US-A- 5 900 247      US-A1- 2002 131 990
US-A1- 2003 228 264

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD

This invention relates to a composition useful for oral care, more particularly care of a dental surface.

### BACKGROUND

It is known to deliver an oral care substance, for example a tooth whitening substance, to a dental surface by means of a removable strip of flexible material having coated thereon a composition such as a gel containing the oral care substance. See for example U.S. Patent No. 5,891,453 and No. 6,096,328, both to Sagel et al.

Another means of delivery of an oral care substance to a dental surface is in a form of a liquid or gel that can be applied to the surface, for example by painting with a soft applicator. For example, on the packaging of Colgate® Simply White® Night clear whitening gel sold by Colgate-Palmolive Co., New York, NY, the user is directed to apply a thin layer of the gel to a dental surface. The gel typically remains in place until removed, for example by toothbrushing.

U.S. Patent Application Publication No. 2003/0124065 discloses compositions said to be useful for dental stain removal and prevention, comprising a vinylpyrrolidone/vinyl acetate copolymer (PVP/VA) having a vinylpyrrolidone/vinyl acetate weight ratio of 30:70 to 90:10, for example 60:40.

US-A-2003/0228264 and WO-A-03/000216 disclose dissolvable tooth-whitening matrixes.

It would be of benefit in the art to have a delivery system for oral care substances in a form of a composition that would remain on a dental surface for a long enough period to be efficacious yet would not have to be mechanically removed at the end of such period.

### SUMMARY

Now provided is a composition, for delivery of an oral care substance to a dental surface upon application of the composition thereto, according to claim 1.

The polymeric matrix includes vinylpyrrolidone (VP) and vinyl acetate (VA) copolymer in a VP/VA weight ratio of about 75:25 to about 20:80. Adjusting the weight ratio of the more hydrophilic VP to the more hydrophobic VA monomers enables dissolution time to be controlled for optimum delivery of the oral care substance.

The composition of the present invention can be used in a method of delivering an oral care substance to a dental surface in a cosmetic tooth-whitening treatment, the method comprising placement of a composition as described above on the surface with sufficient pressure to promote adhesion of the composition to the surface.

### DETAILED DESCRIPTION

A "dental surface" herein is a surface of a natural tooth or a hard surface of artificial dentition including a crown, cap, filling, bridge, denture, dental implant and the like.

An "orally acceptable" compound, composition or vehicle is one that is not harmful to a mammal in amounts disclosed herein when retained in the mouth, without swallowing, for a period sufficient to permit application to a dental surface as required herein. In general, such a compound, composition or vehicle is not harmful even if unintentionally swallowed.

Classification herein of an ingredient as an active or a carrier ingredient is made for clarity and convenience, and no inference should be drawn that a particular ingredient necessarily functions in the composition in accordance with its classification herein. Furthermore, a particular ingredient can serve a plurality of functions, thus disclosure of an ingredient herein as exemplifying one functional class does not exclude the possibility that it can also exemplify another functional class.

Actives useful herein are normally present in the composition in amounts selected to be safe and effective. A "safe and effective" amount in the present context is an amount sufficient to provide a desired benefit, for example a therapeutic, prophylactic or cosmetic effect, when the composition is used repeatedly as described herein, without undue side effects such as toxicity, irritation or allergic reaction, commensurate with a reasonable benefit/risk ratio. Such a safe and effective amount will usually, but not necessarily, fall within ranges approved by appropriate regulatory agencies. A safe and effective amount in a specific case depends on many factors, including the particular benefit desired or condition being treated or sought to be prevented, the particular subject using, or being administered, the composition, the frequency and duration of use, *etc.* Oral care substances are typically present in a total amount of about 0.01% to about 80%, for example about 0.05% to about 60%, about 0.1% to about 50%, or about 0.5% to about 40%, by weight of the composition.

In the present invention the composition comprises as an oral care substance an orally acceptable whitening agent. One or more such agents can be present. Suitable whitening agents include peroxy compounds, chlorine dioxide, chlorites and hypochlorites (*e.g.*, chlorites and hypochlorites of alkali and alkaline earth metals such as lithium, potassium, sodium, magnesium, calcium and barium).

Suitable peroxy compounds include hydrogen peroxide, peroxides of alkali and alkaline earth metals, organic peroxy compounds and peroxy acids and salts thereof. Any orally acceptable compound that delivers a perhydroxy (OOH⁻) ion is useful. A peroxy compound can optionally be present in a form of a polymer-peroxide complex, for example a polyvinylpyrrolidone-hydrogen peroxide (PVP-$H_2O_2$) complex, or encapsulated in a polymer microsphere or nanosphere, for example hydrogen peroxide encapsulated in an allyl methacrylate cross-linked polymer (*e.g.*, Poly-Pore™ of Amcol Health & Beauty Solutions).

Peroxides of alkali and alkaline earth metals include lithium peroxide, potassium peroxide, sodium peroxide, magnesium peroxide, calcium peroxide and barium peroxide.

Organic peroxy compounds include, for example, carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, monoperoxyphthalate and the like.

Peroxy acids and their salts include organic peroxy acids such as alkyl peroxy acids and monoperoxyphthalate, as well as inorganic peroxy acid salts including persulfate, dipersulfate, percarbonate, perphosphate, perborate and persilicate salts of alkali and alkaline earth metals such as lithium, potassium, sodium, magnesium, calcium and barium. Another useful peroxy compound is sodium pyrophosphate peroxyhydrate.

According to the present invention, at least one whitening agent is present in the composition in a total amount effective to result in whitening of a dental surface when applied in accordance with the disclosure herein. Peroxy compounds can illustratively be present in a total hydrogen peroxide equivalent amount of about 0.1% to about 50%, for example about 1% to about 40% or about 5% to about 25%, by weight of the composition. Illustratively, a PVP-$H_2O_2$ complex can be present in an amount of about 5% to about 80% by weight of the composition. Illustratively, sodium percarbonate can be present in an amount of about 0.1% to about 75% by weight of the composition.

The polymeric matrix wherein the oral care substance is dispersed is selected to provide a film that is substantially dissolvable in saliva in a period of time effective for delivery of the oral care substance, when the film is placed in contact with a dental surface. By increasing the proportion of hydrophilic monomers in the polymer matrix, dissolution time of the film can be shortened. Conversely, by increasing the proportion of hydrophobic monomers in the polymer matrix, dissolution time of the film can be lengthened. One of ordinary skill will be able, by routine experimentation based on the disclosure herein, to select suitable hydrophilic and hydrophobic monomers at a suitable weight ratio to provide a dissolution time appropriate for delivery of a particular oral care substance.

Desirably, the dissolution time in saliva is about 5 to about 60 minutes, for example about 5 to about 30 minutes, about 10 to about 30 minutes or about 15 to about 25 minutes. Longer or shorter dissolution times can be useful in specific circumstances.

To provide such dissolution times, the weight ratio of hydrophilic to hydrophobic monomers is 75:25 to 20:80, or preferably 60:40 to 30:70.

The invention is illustrated herein by particular reference to vinylpyrrolidone (VP) as a hydrophilic monomer and vinyl acetate (VA) as a hydrophobic monomer.

In the composition the polymeric matrix comprises a copolymer of vinylpyrrolidone (VP) and vinyl acetate (VA) monomers in a VP/VA weight ratio of 75:25 to 20:80, for example 60:40 to 30:70. Such a copolymer is referred to herein as a poly(vinylpyrrolidone/vinyl acetate) or PVP/VA copolymer. Both random and block copolymers of VP and VA can be useful.

Suitable PVP/VA copolymers typically have an average molecular weight of about 10,000 to about 1,000,000, for example about 20,000 to about 200,000 or about 27,000 to about 70,000.

Illustrative PVP/VA products that can be used include Kollidon® VA 64 of BASF, having a VULVA weight ratio of 60:40, and Luviskol® VA 37E of BASF, having a VP/VA weight ratio of 30:70. PVP/VA products can be supplied as a powder or in a solvent such as ethanol, for example at a concentration of about 30% to about 60%. More than one PVP/VA copolymer can be present in the polymer matrix if desired.

For example, to fine-tune the VP/VA weight ratio in a range from 60:40 to 30:70 for optimum dissolution rate and hence optimum delivery of an oral care substance, a mixture of 60:40 and 30:70 copolymers (e.g., Kollidon® VA 64 and Luviskol® VA 37E respectively) can be prepared in any desired proportion.

A PVP/VA copolymer can be present in an amount of 20% to 65% by weight of the composition. Where the PVP/VA copolymer is the sole film-forming material in the composition, relatively large amounts in the ranges given above will generally be found necessary. Where the PVP/VA copolymer is accompanied by other film-forming polymers, lesser amounts can be useful.

The polymer matrix as a whole (comprising, for example, a PVP/VA copolymer and optionally additional polymeric materials) is present in a film-forming amount, from 30% to 80%, preferably 35% to 65%, by weight of the composition.

Additional polymers in the composition, as a component of or separate from the polymeric matrix wherein the oral care substance is dispersed, can affect such properties of the composition as dissolution rate, adhesiveness to the dental surface, flexibility, mechanical strength, compatibility with the oral care substance, etc.. The additional polymers include PVP and polyethylene oxide, and may optionally further inlude methylcellulose, ethylcellulose, carbomers (carboxyvinyl polymers), polyacrylates etc. The term "polyacrylate" herein encompasses polymers and copolymers having monomeric units selected from acrylic acid, esters and amides and methacrylic acid, esters and amides.

Polyacrylates useful as optional additional polymers are operable to form a film, preferably when cast on a substrate from a solution. In one embodiment, a polyacrylate component comprises a copolymer of one or more first monomeric units selected from the group consisting of acrylic acid, methacrylic acid and combinations thereof, and one or more second monomeric units selected from the group consisting of acrylates, acrylamides and combinations thereof.

Illustratively, the first monomeric units in such a copolymer comprise methacrylic acid or $\beta$-methacrylic acid.

Acrylates useful as second monomeric units in such a copolymer include methyl acrylate, ethyl acrylate, *n*-butyl acrylate, isobutyl acrylate, *t*-butyl acrylate, 2-ethylhexyl acrylate, lauryl acrylate, tridecyl acrylate, cetyl acrylate, stearyl acrylate, cyclohexyl acrylate, benzyl acrylate, isobornyl acrylate, 2-methoxyethyl acrylate, 2-ethoxyethyl acrylate, 2-phenoxyethyl acrylate, tetrahydrofurfuryl acrylate, 2-hydroxyethyl acrylate, 2-hydroxypropyl acrylate, 4-hydroxybutyl acrylate, dimethylaminoethyl acrylate, glycidyl acrylate, allyl acrylate and 1,4-butanediol acrylate. Diacrylate monomers include the diacrylates of 1,4-butanediol, 1,6-hexanediol, tetraethylene glycol, tripropylene glycol and ethoxylated bisphenol-A. Triacrylate monomers include the triacrylates of trimethylol propane, ethoxylated, glyceryl propoxy, and pentaerythritol.

Methacrylates useful as second monomeric units in such a copolymer include methyl methacrylate, ethyl methacrylate, *n*-butyl methacrylate, isobutyl methacrylate, t-butyl methacrylate, 2-ethylhexyl methacrylate, lauryl methacrylate, tridecyl methacrylate, cetyl methacrylate, stearyl methacrylate, cyclohexyl methacrylate, benzyl methacrylate, isobornyl methacrylate, 2-methoxyethyl methacrylate, 2-ethoxyethyl methacrylate, 2-phenoxyethyl methacrylate, tetrahydrofurfuryl methacrylate, 2-hydroxyethyl methacrylate, 2-hydroxypropyl methacrylate, dimethylaminoethyl methacrylate, glycidyl methacrylate, allyl methacrylate, ethylene glycol methacrylate, triethylene glycol methacrylate, tetraethylene glycol methacrylate, 1,3-butyleneglycol methacrylate, 1,6-hexanediol methacrylate, trimethylolpropane methacrylate, ethoxyethyl methacrylate and trifluoroethyl methacrylate.

Acrylamides and methacrylamides useful as second monomeric units in such a copolymer include mono- and di($C_{1-30}$)-alkyl acrylamides and methacrylamides such as those of methyl, ethyl, propyl, butyl, pentyl, hexyl and the like. N-substituted acrylamides can also include N-ethylacrylamide, N-*t*-butylacrylamide, N-*t*-octylacrylamide, N-octylacrylamide, N-decylacrylamide, N-dodecylacrylamide and the corresponding N-substituted methacrylamides. Other N-substituted acrylamides include N-hydroxymethylacrylamide, N-isopropylacrylamide, N-methylacrylamide, N,N'-methylenebisacrylamide, N-isobutoxymethylacrylamide, N,N-dimethylacrylamide and 2-acrylamido-2-methylpropanesulfonic acid.

Suitable polyacrylates illustratively include copolymers of ethyl acrylate and methyl methacrylate (*e.g.*, Kollicoat® EMM 30 D of BASF, poly(ethyl acrylate, methyl methacrylate), 2:1) and copolymers of methacrylic acid and ethyl acrylate (*e.g.*, Kollicoat® MAE 30 DP of BASF, poly(methacrylic acid, ethyl acrylate), 1:1).

Other polyacrylates useful herein include poly(2-hydroxyethyl methacrylate), otherwise known as poly-HEMA, available for example from Polysciences, Inc.; water-soluble acrylic acid copolymers such as MG-0560, MG-0580, and MG-0607 of Dow Coming; and water-swellable polyacrylate polymers such as those of the Eudragit™ series (including E, L, S, RL, RS and NE) of Rohm Pharma, for example Eudragit™ E100.

Polyacrylate copolymers can additionally comprise one or more third monomeric units selected from the group consisting of siloxanes, vinyl esters of $C_{5-12}$ alcohols, and combinations thereof. A siloxane monomer unit includes a silicon-oxygen-silicon bond referred to as a siloxane bond and can be substituted with hydrocarbon radicals attached directly via a carbon atom thereof to the silicon atoms. The most common hydrocarbon radicals are alkyl radicals, especially $C_{1-10}$ alkyl radicals. The resulting silicones can be polymerized or polycondensed. Compounds incorporating the siloxane bond include, but are not limited to, hexamethyldisiloxane, octamethyltrisiloxane, linear siloxanes such as dimethicone and aromatic siloxanes. Siloxane-containing polymers among those useful herein include poly(dimethyl siloxane)-g-polyacrylate polymers, such as Silicone "Plus" Polymer VS80™ of 3M Corporation. Vinyl esters suitable as third monomeric units include vinyl pentanoate, vinyl hexanoate, vinyl heptanoate, vinyl octanoate, vinyl nonanoate, vinyl decanoate and vinyl dodecanoate.

Other optional polymer components comprise salts and esters of acetic acid. A nonlimiting example of such a salt is zinc acetate. Esters include those,with alkyl, aryl and vinyl groups and the like. In one embodiment a polymer component comprises a terpolymer of vinyl acetate, crotonic acid and vinyl neodecanoate (*e.g.*, Luviset™ CAN of BASF). Other terpolymers include a terpolymer of *t*-butyl acrylate, methacrylic acid and dimethicone copolyol (*e.g.*, Luviflex™ Silk of BASF), a terpolymer of N-*t*-butyl acrylamide, ethyl acrylate and acrylic acid (*e.g.*, Ultrahold™ Strong of BASF), and a terpolymer of ethyl acrylate, *t*-butyl acrylate and methacrylic acid (*e.g.*, Luvimer™ of BASF).

Polymers optionally included to enhance adhesiveness are typically alcohol soluble, substantially water insoluble, and stable to the oral care active (*e.g.*, peroxy compound). Such polymers should be substantive (*i.e.*, capable of firm adherence) to teeth or other surfaces of the oral cavity. Such polymers can comprise a calcium complexation moiety and a hydrophobic moiety. Acrylic acid and methacrylic acid monomers can illustratively function as calcium complexation moieties. It is believed, without being bound by theory, that such polymers provide a saliva-resistant film that is secured against a dental surface, for example by inducing additional interaction with enamel of teeth through complexation with $Ca^{2+}$ components thereof, such as hydroxyapatite. Such complexation is believed to provide increased adhesion of the composition to teeth.

If included, additional polymers can be present in the composition, excluding any non-dissolvable backing layer if present, in the following amounts (all percentages by weight):

PVP: about 0.5% to about 70%;
polyethylene oxide: about 1% to about 90%;
methylcellulose: about 0.1% to about 50%;
ethylcellulose: about 0.1% to about 40%;
carbomer: about 0.01% to about 40%;
polyacrylate: about 0.1% to about 50%.

Addition of water-soluble polymers such as PVP or polyethylene oxide provides enhanced adhesion to the dental surface but at the same time result in faster dissolution of the film and thus faster delivery of the oral care substance. On the other hand, addition of less soluble polymers such as methylcellulose, ethylcellulose, carbomer or polyacrylate can retard dissolution of the film. Relative amounts of these various polymers can be adjusted to provide an acceptable balance of properties such as dissolution rate, adhesiveness, flexibility and mechanical strength.

Optionally, a low molecular weight polyethylene glycol (PEG), for example a PEG having an average molecular weight of about 300 to about 1000, such as PEG 600, can be included in the composition, for example as a plasticizer. If included, a low molecular weight PEG can be present in the composition in an amount of about 0.2% to about 40%, for example about 5% to about 20%, by weight.

Alternative plasticizers that can optionally be present include propylene glycol, di- and tripropylene glycols, triacetin, petrolatum, vegetable oils, pegylated (*e.g.*, PEG-40) oils, and the like.

Optionally, one or more natural resins such as rosin (colophony), mastic or shellac can be included in the composition, for example as adhesive agents. If included, such resins can be present in the composition in the following amounts (all percentages by weight):

rosin: about 0.2% to about 40%;
mastic: about 0.5% to about 50%;
shellac: about 0.5% to about 60%.

Optionally, a sweetener can be included in the composition. One or more sweeteners can be present. Use of a sweetener can overcome an unpleasant, for example bitter, taste of an oral care active such as sodium percarbonate. Any orally acceptable natural or artificial, nutritive or non-nutritive sweetener can be used, including without limitation dextrose, polydextrose, sucrose, maltose, dextrin, dried invert sugar, lactose, mannose, xylose, ribose, fructose, galactose, corn syrup (including high fructose corn syrup and corn syrup solids), partially hydrolyzed starch, hydrogenated starch hydrolysate, sorbitol, mannitol, xylitol, maltitol, isomalt, sucralose, aspartame, acesulfame, neotame, D-tryptophan, saccharin and salts thereof (*e.g.*, sodium saccharin), thaumatin, dihydrochalcones, dipeptide-based intense sweeteners, cyclamates (*e.g.*, sodium cyclamate) and the like. One or more sweeteners are optionally present in a total amount depending strongly on the particular sweetener(s) selected, but typically about 0.005% to about 10% by weight of the composition.

In a particular embodiment, the composition comprises sodium saccharin in an amount of about 0.1% to about 5% by weight.

. Optionally, a flavorant can be included in the composition. One or more flavorants can be present. Use of a flavorant, alone or in conjunction with a sweetener, can overcome an unpleasant, for example bitter, taste of an oral care active such as sodium percarbonate. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc*., bean- and nut-derived flavors such as coffee, cocoa, cola, peanut, almond, *etc*., adsorbed and encapsulated flavorants and the like. Also encompassed within flavorants herein are ingredients that provide fragrance and/or other sensory effect in the mouth, including cooling or warning effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. If included, one or more flavorants can be present in a total amount of about 0.01% to about 5%, for example about 0.1% to about 2.5% by weight of the composition.

Optionally, a colorant can be included in the composition. One or more colorants can be present. Colorants herein include pigments, dyes, lakes and agents imparting a particular luster or reflectivity such as pearling agents. A colorant can serve a number of functions, including for example to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including without limitation talc, mica, magnesium carbonate, calcium carbonate, magnesium silicate, magnesium aluminum silicate, silica, alumina, hydroxyapatite, titanium dioxide, zinc oxide, red, yellow, brown and black iron oxides, ferric ammonium ferrocyanide, manganese violet, ultramarine titaniated mica, bismuth oxychloride and the like. If included, one or more colorants can be present in a total amount of about 0.001% to about 20%, for example about 0.01% to about 10% or about 0.1% to about 5% by weight of the composition. Colorants such as alumina, hydroxyapatite and titanium dioxide that provide an opaque white appearance to the film can be especially desirable in matching appearance of teeth.

Oral care actives or other ingredients can optionally be present in the film composition in encapsulated form. For example, peroxy compounds, flavor and/or color ingredients can be encapsulated. Encapsulation can enhance stability of such ingredients upon drying of the film, but permit release of the ingredients upon placement in the humid environment of the oral cavity. Further, encapsulation can protect incompatible ingredients from one another. Encapsulation can also provide an additional means of control of release rate and/or time of an oral care active.

As described below, solvent materials such as ethanol, ethyl acetate and water can to a large extent be removed from the composition during a process of manufacture, but some amount of solvent can remain in the finished film. Amount of organic solvent such as ethanol or ethyl acetate can be zero to about 15%, more typically zero to about 5%, by weight of the film composition. Amount of water can be zero to about 20%, more typically zero to about 5%, by weight of the film composition.

The composition further comprises a backing layer that is non-dissolvable in saliva. Such a backing layer typically comprises one or more water-insoluble polymers such as ethylcellulose, polyethylene or PET (polyethylene terephthalate).

A dissolvable film useful according to the invention can be prepared by any process known in the art for making polymer-based films, such as for example a casting process. In an illustrative process, the materials to be included in the polymeric matrix (for example a PVP/VA copolymer and optionally other polymers) are first added to a suitable amount of a solvent or mixture of solvents in a vessel and stirred until dissolved or homogeneously dispersed. Suitable solvents include water, ethanol and ethyl acetate. The oral care substance(s) and optionally other ingredients such as sweetener are then added with stirring until dissolved or homogeneously dispersed. The resulting liquid is then cast on a heated surface, where the composition forms a film upon partial or complete evaporation of the solvent or solvent mixture.

The dental surface to which the composition is applied can be in a human or nonhuman subject, for example a nonhuman mammalian subject such as a companion animal, for example a dog or cat. The dental surface may be a surface of one or more natural teeth, but the composition may also be applied to a surface of artificial dentition, for example a crown, a cap, a filling, a bridge, a denture or a dental implant.

A method of whitening a dental surface may comprise placing on the surface a film composition of the invention that comprises a whitening agent as described above, wherein during the placing sufficient pressure is applied to promote adhesion of the film to the surface.

Degree of whitening of a dental surface can be observed visually, for example with the aid of color comparison charts, gauges or shade guides, *e.g.*, as described by Browning (2003), Journal of Esthetic Restorative Dentistry 15 Supp. 1, S13-S20, incorporated herein by reference.

Alternatively, staining or inhibition thereof can be measured by colorimetry, using any suitable instrument such as a Minolta Chromameter, *e.g.*, model CR-321 (Minolta Corp., Ramsey, NJ). The instrument can be programmed, for example, to measure Hunter Lab values or L*a*b* values according to the standard established by the International Committee of Illumination (CIE). The L*a*b* system provides a numerical representation of three-dimensional color space where L* represents a lightness axis, a* represents a red-green axis and b* represents a yellow-blue axis. The L* and b* axes are typically of greatest applicability to tooth whitening, which can be measured as increase in whiteness relative to an untreated surface. Increase in whiteness can be computed from differences in L*, a* and b* values between untreated and treated surfaces. A useful parameter is ΔE*, calculated as the square root of the sum of the squares of differences in L*, as and b* values, using the formula:

$$\Delta E^* = [(\Delta L^*)^2 + (\Delta a^*)^2 + (\Delta b^*)^2]^{1/2}$$

A higher value of $\Delta E^*$ indicates greater increase in whiteness.

The film may be left in place until it has substantially dissolved in saliva. The time taken for this dissolution to occur varies depending on the precise composition of the film. If the film dissolves too rapidly, the oral carve substance may be inadequately delivered to the dental surface, instead being to a considerable extent removed in saliva from the site of placement of the film. The film should therefore be selected to dissolve in a time period effective to deliver the oral care substance to the dental surface, for example a period of about 5 to about 60 minutes, about 5 to about 30 minutes or about 10 to about 30 minutes as indicated above.

If desired, remnants of the film can later be removed, for example by brushing. In some embodiments, however, the film dissolves so completely that no residue remains for later removal.

Practice of the method can consist of a single application as described herein, or can comprise repeated such applications. In one embodiment the method is repeated at regular intervals, for example twice or once daily, twice or once weekly, twice or once monthly, in a program or regimen conducted at home and/or in a professional or clinical setting.

The invention can further be understood by reference to the following nonlimiting examples.

## EXAMPLES

### Example 1

Film compositions A-F were prepared having the ingredients shown in Table 1. The compositions were prepared by placing a suitable amount of solvent (ethanol and/or ethyl acetate) in a beaker and adding the polymer components to the solvent with stirring until dissolved or dispersed. The oral care substance (in the present example sodium percarbonate or PVP-$H_2O_2$) and sodium saccharin were added and stirred for 10 minutes, followed by casting of the resulting mixture at 80°C for 6-15 minutes to provide a film.

Table 1: Film compositions of Example 1

| Ingredient | Weight % | | | | | |
|---|---|---|---|---|---|---|
| | A | B | C | D | E | F |
| ethanol | 2.80 | | 2.02 | 1.55 | 3.50 | 2.00 |
| ethyl acetate | | 2.80 | | | 2.10 | 2.00 |
| water | | | | | 49.05 | |
| PVP/VA copolymer | 23.52 | 23.52 | 44.41 | 61.97 | 49.05 | 40.00 |
| polyvinylpyrrolidone | 23.52 | 23.52 | | | | |
| Lpolyethylene oxide | | | 10.09 | | 9.81 | |
| PEG 600 | 14.56 | 14.56 | 8.07 | 8.85 | 9.81 | 10.00 |
| rosin (colophony) | | | | | | 5.00 |
| mastic | | | | | | 5.00 |
| shellac | | | | | | 5.00 |
| hydroxyapatite | 10.08 | 10.08 | 10.09 | | | 5.00 |
| PVP-$H_2O_2$ | | | | 26.54 | | |
| sodium percarbonate | 25.20 | 25.20 | 25.03 | | 25.23 | 25.00 |
| sodium saccharin | 0.16 | 0.16 | 0.30 | 1.00 | 0.50 | 1.00 |

In compositions A to F, the particular PVPNA copolymer used was Luviskol® VA 37E of BASF, having a VPNA weight ratio of 30:70. Other PVP/VA copolymers could be substituted if desired.

### Example 2

Effectiveness of film composition A of Example 1 in whitening extracted human teeth was tested. Degree of whitening was measured on the L*a*b* system as established by CIE using a Minolta CR-321 chromameter before and after treatment. Increase in whiteness was determined as $\Delta E$, a higher value of $AE^*$ indicating a greater increase in whiteness. After 14 successive treatments with the film, $\Delta E^*$ was 3.4, indicating that the teeth were becoming whiter.

## Claims

1. A composition for delivery of an oral care substance to a dental surface upon application of the composition thereto, the composition consisting of a flexible film and a backing layer that is non-dissolvable in saliva, said backing layer comprising one or more water in soluble polymers, the flexible film comprising the oral care substance dispersed in a film-forming effective amount of a polymeric matrix having a hydrophilic component and a hydrophobic component in a weight ratio selected such that the film is substantially dissolvable in saliva in a period of time effective for delivery of the oral care substance, wherein the oral care substance includes a whitening agent, and the polymeric matrix comprises from 30 to 80% by weight of the composition, the polymer matrix including a poly(vinylpyrrolidone/vinyl acetate) copolymer in which the vinylpyrrolidone to vinyl acetate weight ratio in the copolymer is 75:25 to 20:80 and the poly(vinylpyrrolidone/vinyl acetate) copolymer is present in an amount of 20% to 65% by weight of the composition, and at least one polymer selected from polyvinylpyrrolidone and polyethylene oxide.

2. The composition of Claim 1 wherein the vinylpyrrolidone to vinyl acetate weight ratio in the copolymer is 60:40 to 30:70.

3. The composition of Claim 1 wherein the polymer matrix further comprises at least one polymer selected from methylcellulose, ethylcellulose, carbomers and polyacrylates.

4. The composition of Claim 1 further comprising at least one natural resin.

5. The composition of Claim 1 further comprising a sweetener.

6. The composition of Claim 1 wherein the film is substantially dissolvable in saliva in 5 to 60 minutes.

7. The composition of Claim 1 wherein the film is substantially dissolvable in saliva in 5 to 3 0 minutes.

8. The composition oaf Claim 1 wherein the film is substantially dissolvable in saliva in 10 to 30 minutes.

9. The composition of Claim 1 wherein the whitening agent is selected from peroxy compounds, chlorine dioxide, chlorites and hypochlorites.

10. The composition of Claim 9 wherein the peroxy compound is selected from hydrogen peroxide, peroxides of alkali and alkaline earth metals, organic peroxy compounds, peroxy acids and salts thereof, and polymer-peroxide complexes.

11. The composition of Claim 10 wherein the peroxy compound is sodium percarbonate.

12. The composition of Claim 1 wherein the whitening agent is at least one peroxy compound in a total hydrogen peroxide equivalent amount of 0.1% to 50% by weight.

## Patentansprüche

1. Zusammensetzung zur Abgabe einer Mundpflegesubstanz auf eine Dentaloberfläche bei Verabreichung der Zusammensetzung darauf, wobei die Zusammensetzung aus einem flexiblen Film und einer Unterstützungsschicht besteht, die in Speichel nicht löslich ist, wobei die Unterstützungsschicht ein oder mehrere wasserunlösliche Polymere umfasst, und wobei der flexible Film eine Mundpflegesubstanz umfasst, die in einer zur Filmbildung effektiven Menge einer Polymermatrix mit einer hydrophilen Komponente und einer hydrophoben Komponente in einem solchen Gewichtsverhältnis, dass der Film im Wesentlichen in einer zur Abgabe der Mundpflegesubstanz effektiven Zeitspanne in Speichel auflösbar ist, wobei die Mundpflegesubstanz ein Weißungsmittel enthält und die Polymermatrix 30 bis 80 Gew.% der Zusammensetzung ausmacht, wobei die Polymermatrix ein Poly(vinylpyrrolidon/vinylacetat)-Copolymer, in dem das Gewichtsverhältnis von Vinylpyrrolidin zu Vinylacetat im Copolymer 75:25 bis 20:80 beträgt, und das Poly(vinylpyrrolidon/vinylacetat)-Copolymer in einer Menge von 20 bis 65 Gew.% der Zusammensetzung vorhanden ist, und mindestens ein Polymer enthält, das aus Polyvinylpyrrolidon und Polyethylenoxid ausgewählt ist.

**2.** Zusammensetzung nach Anspruch 1, wobei das Gewichtsverhältnis von Vinylpyrrolidon zu Vinylacetat im Copolymer 60:40 bis 30:70 beträgt.

**3.** Zusammensetzung nach Anspruch 1, wobei die Polymermatrix weiterhin mindestens ein Polymer umfasst, dass aus Methylcellulose, Ethylcellulose, Carbomeren und Polyacrylaten ausgewählt ist.

**4.** Zusammensetzung nach Anspruch 1, die weiterhin mindestens ein natürliches Harz umfasst.

**5.** Zusammensetzung nach Anspruch 1, die weiterhin ein Süßungsmittel umfasst.

**6.** Zusammensetzung nach Anspruch 1, wobei der Film im Wesentlichen in 5 bis 60 Minuten in Speichel auflösbar ist.

**7.** Zusammensetzung nach Anspruch 1, wobei der Film im Wesentlichen in 5 bis 30 Minuten in Speichel auflösbar ist.

**8.** Zusammensetzung nach Anspruch 1, wobei der Film im Wesentlichen in 10 bis 30 Minuten in Speichel auflösbar ist.

**9.** Zusammensetzung nach Anspruch 1, wobei das Weißungsmittel aus Peroxyverbindungen, Chlordioxid, Chloriten und Hypochloriten ausgewählt ist.

**10.** Zusammensetzung nach Anspruch 9, wobei die Peroxyverbindung aus Wasserstoffperoxid, Peroxiden von Alkali- und Erdalkalimetallen, organischen Peroxyverbindungen, Peroxysäure und Salzen davon und Polymer-Peroxid-Komplexen ausgewählt ist.

**11.** Zusammensetzung nach Anspruch 10, wobei die Peroxyverbindung Natriumpercarbonat ist.

**12.** Zusammensetzung nach Anspruch 1, wobei das Weißungsmittel mindestens eine Peroxyverbindung in einer Wasserstoffperoxid-äquivalenten Gesamtmenge von 0,1 bis 50 Gew.% ist.

**Revendications**

**1.** Composition pour la délivrance d'une substance de soin oral à une surface dentaire lors de l'application de la composition sur celle-ci, la composition étant constituée d'un film flexible et d'une couche arrière qui est non soluble dans la salive, ladite couche arrière comprenant un ou plusieurs polymères insolubles dans l'eau, le film flexible comprenant la substance de soin oral dispersée dans une quantité filmogène efficace d'une matrice polymère comprenant un composant hydrophile et un composant hydrophobe en un rapport en poids choisi de manière à ce que le film soit essentiellement soluble dans la salive en une durée efficace pour la délivrance de la substance de soin oral, la substance de soin oral comprenant un agent de blanchiment, et la matrice polymère représentant de 30 à 80 % en poids de la composition, la matrice polymère comprenant un copolymère poly(vinylpyrrolidone/acétate de vinyle), le rapport en poids entre la vinylpyrrolidone et l'acétate de vinyle dans le copolymère étant de 75:25 à 20:80 et le copolymère poly(vinylpyrrolidone/acétate de vinyle) étant présent en une quantité de 20 % à 65 % en poids de la composition, et au moins un polymère choisi parmi la polyvinylpyrrolidone et l'oxyde de polyéthylène.

**2.** Composition selon la revendication 1, dans laquelle le rapport en poids entre la vinylyrrolidone et l'acétate de vinyle dans le copolymère est de 60:40 à 30:70.

**3.** Composition selon la revendication 1, dans laquelle la matrice polymère comprend en outre au moins un polymère choisi parmi la méthylcellulose, l'éthylcellulose, les carbomères et les polyacrylates.

**4.** Composition selon la revendication 1, comprenant en outre au moins une résine naturelle.

**5.** Composition selon la revendication 1, comprenant en outre un édulcorant.

**6.** Composition selon la revendication 1, dans laquelle le film est essentiellement soluble dans la salive en 5 à 60 minutes.

**7.** Composition selon la revendication 1, dans laquelle le film est essentiellement soluble dans la salive en 5 à 30 minutes.

8. Composition selon la revendication 1, dans laquelle le film est essentiellement soluble dans la salive en 10 à 30 minutes.

9. Composition selon la revendication 1, dans laquelle l'agent de blanchiment est choisi parmi les composés peroxy, le dioxyde de chlore, les chlorites et les hypochlorites.

10. Composition selon la revendication 9, dans laquelle le composé peroxy est choisi parmi le peroxyde d'hydrogène, les peroxydes de métaux alcalins et alcalino-terreux, les composés peroxy organiques, les peroxyacides et leurs sels, et les complexes polymère-peroxyde.

11. Composition selon la revendication 10, dans laquelle le composé peroxy est le percarbonate de sodium.

12. Composition selon la revendication 1, dans laquelle l'agent de blanchiment est au moins un composé peroxy en une quantité équivalente de peroxyde d'hydrogène totale de 0,1 % à 50 % en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5891453 A
- US 6096328 A, Sagel
- US 20030124065 A
- US 20030228264 A
- WO 03000216 A


**Non-patent literature cited in the description**

- **BROWNING.** *Journal of Esthetic Restorative Dentistry,* 2003, vol. 15 (1), S13-S20